# FASCICULE DE BREVET EUROPEEN

(11) **EP 0 827 427 B1**
(45) Date de publication et mention de la délivrance du brevet: **23.08.2000**
(21) Numéro de dépôt: 96917535.5
(22) Date de dépôt: 23.05.1996
(51) Int. Cl.: B01L 9/06, G01N 33/80

(54) **DISPOSITIF ET PROCEDE POUR TESTS ET ANALYSES DE FLUIDES**
VORRICHTUNG UND VERFAHREN ZUR UNTERSUCHUNG VON FLÜSSIGKEITEN
FLUID TESTING AND ANALYSING DEVICE AND METHOD

(30) Priorité: 23.05.1995 FR 9506140
(43) Date de publication de la demande: 11.03.1998
(73) Titulaire: Dubus, Yves Francois Pierre, 33120 Arcachon (FR)
(72) Inventeur: Dubus, Yves Francois Pierre, 33120 Arcachon (FR)
(74) Mandataire: Leboyer, Jean-Jacques
(86) Numéro de dépôt international: FR9600770
(87) Numéro de publication internationale: WO9637304

(56) Documents cités:
- WO-A-85/05039
- WO-A-86/03008
- WO-A-87/01461
- WO-A-92/08988
- US-A- 4 058 363
- US-A- 5 140 993

## Description

La présente invention concerne un dispositif et un procédé pour la réalisation de tests et/ou d'analyses de fluides, notamment en vue de la détermination de groupes sanguins ou de tests de compatibilité. Elle concerne en particulier un dispositif et un procédé permettant la réalisation de tests et d'analyses de fluides dans des conditions de simplicité et de sécurité qui les rendent particulièrement attractifs.

Ce dispositif et ce procédé sont particulièrement adaptés pour la réalisation de tests et d'analyses en l'absence de contamination par et vers l'environnement.

L'invention concerne tout particulièrement, mais non exclusivement, un dispositif et un procédé pour la détermination des groupes sanguins, notamment en relation avec une transfusion sanguine, par test visuel en présence d'un réactif.

On connaît déjà plusieurs types de dispositifs pour la détermination du groupe sanguin d'un patient ou la réalisation de tests de compatibilité sanguine.

C'est ainsi que le document EP-A-0 054 087 décrit un appareil pour tester des fluides déposés en couches sur supports, apte à imprimer une force centrifuge aux dits fluides, auxquels est appliqué un anticorps approprié, pour une révélation de compatibilité antigène-anticorps. Cet appareil est de fabrication et de maniement complexes. Il nécessite un asservissement à une source d'énergie extérieure.

Le document EP-A-0 104 881 décrit un dispositif pour tests de compatibilité transfusionnelle destiné à être utilisé au lit d'un patient auquel une transfusion doit être pratiquée. Ce dispositif peut être fixé à la poche de sang et reçoit par capillarité une quantité spécifiée du sang du patient, mis ensuite en relation avec un antisérum. La pièce plastique poreuse dans laquelle cette capillarité s'exerce doit être telle que seul le sang non-agglutiné puisse continuer à migrer par capillarité. Une telle pièce, qui est constituée d'un matériau poreux absorbant, n'est pas protégée avant d'être introduite dans l'antisérum révélateur de groupe sanguin.

Le document FR-A-2 586 815 décrit un dispositif de détermination d'un groupe sanguin comportant, pour l'examen visuel d'un mélange d'un échantillon de sang et d'un sérum-test, au moins une chambre fermée et des moyens pour introduire l'échantillon de sang dans cette chambre. Des moyens sont prévus pour évacuer l'air de la chambre à travers une membrane perméable hydrophobe, afin de permettre à l'échantillon d'y pénétrer sans que s'y oppose une surpression d'air. L'injection de sang du receveur s'effectue au moyen d'une seringue.

Le document WO-A-85 05039 décrit un porte-tubes à essai comportant un système de mise sous vide, dans lequel des tubes contenant des échantillons à tester comportent un bouchage à leur extrémité supérieure et dans leur fond un bouchon élastique destiné à être perforé par une aiguille creuse. Un vide partiel est ensuite établi dans les tubes. Le contenu de chaque tube est ainsi extrait en une fois, dans sa totalité, sans possibilité d'extractions partielles ou répétées.

Le document WO-A-92 08988 divulgue un appareillage complexe et automatisé pour les prises d'essais de liquides et le nettoyage simultané du système de prélèvement de prises d'essais. Ce système comporte une station de chargement de sérum, une station de détection, un tableau d'indexation pour régler l'avancement des tubes vers une station de collecte, une station de prélèvement de parties aliquotes et une station de lavage. Là encore le prélèvement s'opère par l'intermédiaire d'une aiguille dont la pointe est introduite dans le tube concerné et un vide est ensuite appliqué à l'aiguille, par laquelle le liquide est entièrement extrait du tube.

Le document WO-A-87 01461 décrit un dispositif pour la détermination d'un groupe sanguin, dans lequel des moyens permettent d'introduire un échantillon de sang à tester et/ou le sérum-test dans une chambre fermée où s'effectue l'observation de la réaction du sérum-test avec le sang. En pratique, un organe mobile tel qu'une aiguille creuse assure le transfert dans la chambre d'une quantité de sang déterminée par le degré de vide partiel ou dépression préalablement établi dans ladite chambre. Plus préférentiellement, le dispositif comprend un boîtier en deux pièces déplaçables l'une par rapport à l'autre et chacune par rapport à l'aiguille, qui a alors deux pointes d'extrémités perforant respectivement et successivement la paroi du récipient de prélèvement et celle de la chambre.

Aucun des dispositifs de test de la technique antérieure ne satisfait pleinement à l'ensemble des conditions actuellement exigées pour de tels dispositifs, telles que des conditions de fiabilité, de maniabilité et de sécurité optimisées, en particulier dans le domaine de la détermination des groupes sanguins, notamment lors de transfusions de sang.

Les problèmes le plus souvent rencontrés lors de tests de contrôle et d'analyse en tous domaines sont en effet les risques de pollution de la substance à analyser et ceux de souillure ou de contamination pour le manipulateur. D'autre part, l'absence de possibilité de conservation des réactions dans le temps empêche de stocker des résultats précédemment obtenus.

Plus particulièrement dans le domaine médical, il peut être parfois malaisé, voire dangereux pour le manipulateur en raison de contaminations virales possibles, de pratiquer juste avant une transfusion un contrôle de compatibilité sanguine, qui consiste à vérifier la corrélation entre le sang du malade (receveur) et celui contenu dans la poche de sang (donneur), destiné à être transfusé au malade. Les dispositifs actuellement disponibles n'apportent pas tous les avantages attendus à cet égard, dans la pratique. Ils ne procurent pas non plus une totale sécurité quant à l'affectation des résultats et à leur suivi.

Il existait donc un besoin pour un dispositif fiable, sûr et aisé à manipuler pour la réalisation de tests de contrôle et d'analyses, utilisable en tous domaines et notamment en biologie humaine et vétérinaire.

On a maintenant trouvé de manière inattendue que ces avantages, ainsi que d'autres qui ressortiront mieux à la lecture de la description ci-après, sont satisfaits par un dispositif et un procédé selon la présente invention.

La présente invention vise à fournir un tel dispositif et un procédé pour sa mise en oeuvre, permettant de mieux satisfaire aux exigences légitimes des utilisateurs et apportant à ceux-ci aussi bien un confort d'utilisation qu'une plus grande tranquillité dans l'obtention de résultats non perturbés par des causes externes et en toute sécurité.

La présente invention a également pour objectif de procurer un dispositif et un procédé de test et d'analyse exemptant l'utilisateur de manipulations complexes et étendant par conséquent le champ de son utilisation à toutes personnes, même non spécialement formées, et en tous lieux.

Un autre objectif de la présente invention est de procurer un tel dispositif, pour lequel il n'est pas nécessaire de recourir à un vide ou mise sous dépression de la chambre de test et de visualisation.

Il est effet apparu que le dispositif selon l'invention procure, de façon inattendue, une simplicité, une sécurité et une fiabilité d'utilisation qui ne pouvaient être atteintes jusqu'ici, et ce même lors d'utilisations par des personnes non spécialement formées.

L'invention a pour premier objet un dispositif pour la réalisation de tests et/ou d'analyses de fluides, tel que défini dans la revendication 1. Des modes de réalisation préférés sont définis dans les revendications dépendantes.

Pour l'utilisation de boudins souples, le dispositif est avantageusement complété par un pièce formant portoir pour leur introduction sur au moin une aiguille de façon à permettre leur perçage non traversant par au moins une aiguille susdite.

Selon une forme de réalisation préférée, le dispositif selon l'invention comporte un porte-tubes combiné avec au moins une pièce formant portoir pour l'utilisation combinée de tubes d'échantillon et de boudins souples de prises d'essai.

L'invention a également pour objet un procédé pour la réalisation de tests et/ou d'analyses de fluides, tel que défini dans la revendication 7.

L'invention a enfin pour objet l'utilisation d'un dispositif de l'invention en biologie humaine ou vétérinaire.

L'invention est décrite plus concrètement ci-après en référence aux dessins annexés, qui ne la limitent aucunement et dans lesquels:

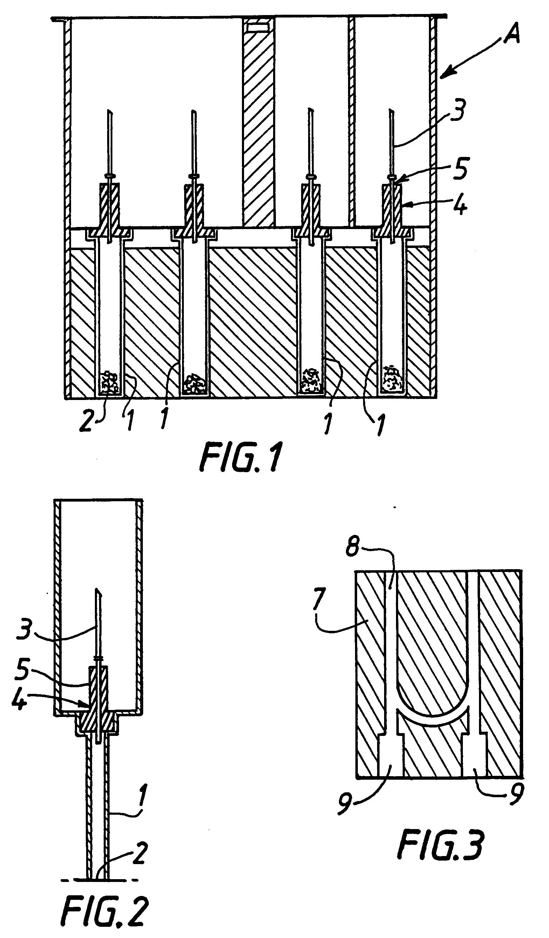

- Fig. 1 représente une vue en coupe schématique longitudinale selon un plan vertical médian d'une forme de réalisation du dispositif selon l'invention, posé debout;
- Fig. 2 est une vue en coupe transversale schématique du dispositif selon la figure 1;
- Fig. 3 est une vue en coupe schématique partielle d'un portoir pour tube souple;
- Fig. 4 est une vue en coupe transversale schématique selon un plan vertical médian du dispositif selon la figure 1 en position debout;
- Fig. 5 est une vue en coupe schématique partielle d'une variante de portoir, encliquetée sur un dispositif de test et/ou d'analyse selon la figure 1, et dont seule la partie supérieure est représentée.

Pour plus de commodité, l'invention sera décrite dans la suite en référence le plus souvent à une telle utilisation. Cependant il doit être entendu que cette application particulière ne fait qu'illustrer plus précisément l'invention et ne limite aucunement la portée de celle-ci.

Le dispositif selon l'invention comprend un porte-tubes A permettant de maintenir en position debout au moins un tube 1, qui peut déjà contenir ou non un réactif 2 approprié pour le test à effectuer. Le réactif peut être préalablement introduit dans le tube avant l'obturation de celui-ci. Ce doit être le cas si le dit réactif est solide.

Le porte-tubes A peut être réalisé en tous matériaux, de préférence en matière plastique, et il doit laisser visibles au moins des portions de paroi des tubes qu'il porte. Dans la pratique cela peut être obtenu soit par la transparence propre du matériau choisi pour le dit porte-tubes, soit par l'absence de paroi du porte-tubes au droit des portions de paroi de tubes qui doivent satisfaire à cette condition. Le porte-tubes et/ou les tubes peuvent être par exemple, en totalité ou en partie, en verre ou en matière plastique telle que du polypropylène, du polycarbonate, du polyéthylène ou autres.

Les tubes 1 peuvent être introduits dans des logements appropriés du porte-tubes ou faire partie intégrante de celui-ci, avantageusement sous forme d'alvéoles ménagées dans le porte-tubes lui-même. Ils peuvent avoir une section droite quelconque, par exemple carrée, triangulaire, circulaire, polygonale ou autre. Une section transversale ovoïde est préférée, pour ne pas créer de problème de capillarité et d'adhérence électrostatique des réactifs et des produits réactionnels aux angles d'une section comportant des angles droits ou aigus.

On préfère des tubes de faible section, où un faible volume de réactif suffit. La hauteur du tube facilite le "chaloupage" du mélange réactif lorsque l'on imprime au dispositif un mouvement de basculement alterné et ainsi optimise la lecture de la réaction.

L'orifice supérieur du tube 1 est traversé en son centre par une aiguille 3 rendue solidaire d'un moyen de bouchage 4 approprié pour obturer l'orifice du dit tube 1. Ce moyen de bouchage peut être en tous matériaux classiques pour cette fonction; il doit présenter une rigidité suffisante. Selon une forme de réalisation préférée, l'ensemble de l'aiguille et du moyen de bouchage, ainsi qu'éventuellement de la partie centrale surélevée ou collerette 5 surmontant celui-ci, peut être obtenu par des techniques de surmoulage connues de l'homme du métier. En variante, la dite collerette peut être rapportée et être laissée en coulissement libre sur l'aiguille, ou de préférence être fixée de manière adhérente à celle-ci ou à la face du bouchon tournée vers la pointe de l'aiguille.

La rehausse susdite peut être réalisée par un prolongement approprié, vers le haut en position d'utilisation, des parois du porte-tubes, dans le prolongement direct de celles-ci ou en décalage par rapport à elles. Elle est avantageusement profilée de manière à, ou comporte sur ses parois tournées vers l'intérieur du dispositif des moyens pour, assurer un guidage des tubes 6 et/ou des portoirs 7 comportant des échantillons à tester.

Le portoir 7 est de préférence constitué par un bloc de dimensions appropriées pour son introduction telle que décrite ci-dessus et comportant, en position d'utilisation, un canal 8 soit en U débouchant vers le haut, soit sensiblement linéaire débouchant latéralement, ainsi qu'au moins un alésage non traversant 9 et sensiblement vertical, mettant en communication le dit canal et l'extérieur sur la face inférieure du portoir.

Selon une forme de réalisation avantageuse, le portoir 7 comporte au moins deux alésages et il comprend en outre, en option, des moyens pour effectuer des pressions sur le boudin souple 10, une fois celui-ci introduit dans le portoir, et des moyens 12 pour encliqueter le portoir dans sa position d'utilisation et de préférence pour empêcher qu'il soit enlevé après réalisation du test, par exemple sous la forme d'ergots anti-retour dans ou à proximité des guides servant à son introduction dans le porte-tubes.

Pour parfaire encore la sécurité du dispositif au regard des risques de souillure ou de contamination, ainsi que pour supprimer les risques d'évaporation du réactif avant utilisation, il est avantageux de munir la portion des aiguilles extérieures aux tubes de tests d'une gaine perforable 11, étanche aux liquides et de préférence aussi aux gaz, par exemple en matière plastique souple telle que du poly(chlorure de vinyle) ou en latex de caoutchouc. Cette gaine, qui a en pratique la forme générale d'un préservatif, est destinée à être perforée par l'aiguille et se trouve ensuite repliée en accordéon entre la base de l'aiguille et le bouchon du tube de prélèvement d'échantillon, où elle contribue à parfaire l'étanchéité, si nécessaire.

En ce qui concerne plus particulièrement le domaine des tests et analyses en biologie humaine et vétérinaire, il s'est avéré que le dispositif selon l'invention est tout spécialement approprié, ainsi qu'il sera montré plus loin, pour être utilisé en combinaison avec les tubes à prélèvement classiques de tous types, dont le système de bouchage présente les caractéristiques de perforabilité et de souplesse requises.

Pour utiliser le dispositif selon l'invention, on appuie un tube bouché comportant un prélèvement d'échantillon, en position renversée sur la pointe d'une aiguille de l'un des tubes du porte-tubes et on exerce une pression verticale descendante de façon à faire déformer le bouchon souple de ce tube par la collerette susdite, pour exercer dans celui-ci une pression de fluide suffisante pour faire sortir du dit tube une ou plusieurs gouttes de l'échantillon. Cette opération peut être répétée autant de fois que nécessaire. De même, si le prélèvement d'échantillon est contenu dans un boudin souple, celui-ci est introduit dans un bloc portoir tel que décrit plus haut, qui est lui-même amené en regard d'au moins une aiguille pour permettre une perforation non traversante du boudin par la dite aiguille et on exerce au moins une pression du boudin, soit directement par pression manuelle ou assistée sur au moins l'une des extrémités de celui-ci dépassant du portoir, soit par l'intermédiaire d'un organe de pressage intégré au portoir, soit encore par une presion verticale descendante sur le portoir lui-même, et ce autant de fois que nécessaire. Là encore, chaque pression exercée sur le boudin a pour effet de forcer au moins une goutte de fluide échantillon à pénétrer par le canal de l'aiguille dans le tube correspondant formant chambre fermée de réaction.

La réaction révélant le résultat du test est de type connu. Elle est le résultat d'une modification du mélange entre le réactif contenu ou préalablement introduit dans la chambre réactionnelle et l'échantillon à analyser, par exemple du sang dont on détermine ou contrôle ainsi le groupe sanguin. Elle fait appel à des réactifs soit solides, soit liquides, éventuellement sur support, qui sont aptes à déclencher en présence d'un échantillon répondant au test soit une réaction colorée, soit une agglutination, soit encore d'autres types de réactions visualisables par l'oeil humain et/ou par des appareils d'évaluation, comme par exemple un trouble mesurable par turbidimétrie ou une hémolyse. En pratique le dispositif selon l'invention est destiné à être utilisé pour une visualisation des résultats des tests directement par l'oeil.

L'identification des prélèvements testés est facilitée selon l'invention par la possibilité qu'offre le porte-tubes de recevoir sur au moins certaines de ses surfaces visibles des indications, claires ou codées, appropriées pour permettre un suivi parfait et une sécurité renforcée des tests. Ce dispositif peut en outre, si on le souhaite, être connecté à un système de reconnaissance automatisée, avantageusement sur critères numérisés.

Selon une variante préférée pour des applications médicales, en particulier pour la transfusion sanguine, ce dispositif peut recevoir une carte ou autre support muni d'une puce, sur laquelle peuvent être mémorisés préalablement par des moyens appropriés l'empreinte digitale, ainsi que d'autres informations concernant le patient à transfuser et/ou des informations concernant la poche de sang à transfuser.

Le dispositif selon l'invention peut être utilisé pour tous types de tests, notament mais non exclusivement en biologie humaine ou vétérinaire, en particulier pour des tests virologiques (par exemple VIH, entre autres) ou bactériologiques (par exemple sur sondage urinaire ou ponction de liquide céphalo-rachidien).

Les tests ainsi effectués répondent bien aux exigences d'instantanéité et de rapidité des résultats, tout en procurant au manipulateur une garantie d'absence de risque de contamination. Les réactions mises en oeuvre peuvent alors être des réactions d'hémagglutination, d'agglutination au latex ou tout autre type de réaction interprétable à l'oeil nu.

La simplicité d'utilisation de ce dispositif, prêt à l'emploi, permet qu'il soit utilisé par toutes personnes, même sans connaissance particulière en biologie, notamment par tous médecins hospitaliers, médecins de ville, infirmières, malades eux-mêmes ou simples particuliers.

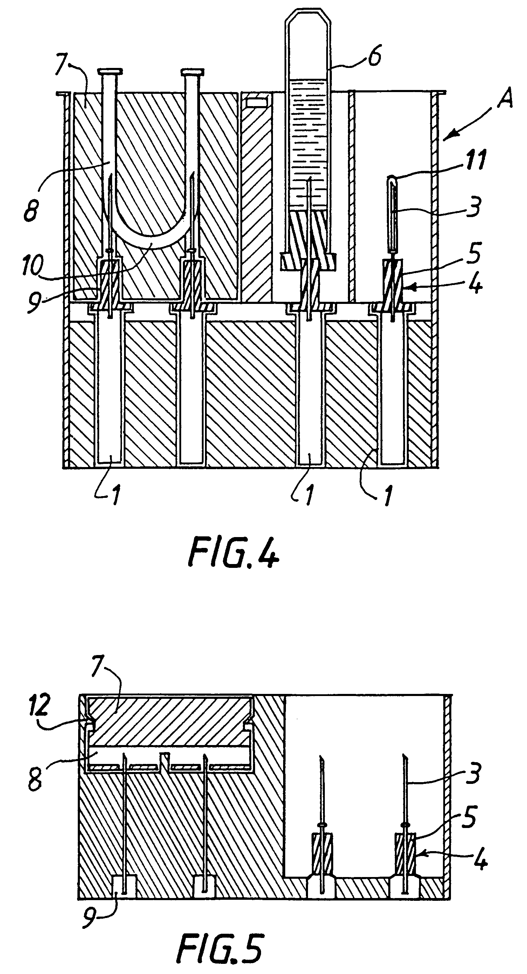

Un exemple illustratif d'une application dans ce domaine est donné ci-après, en référence à la figure 4 des dessins annexés.

On a utilisé le dispositif selon l'invention correspondant à cette variante de réalisation pour pratiquer, immédiatement avant une transfusion, un contrôle de compatibilité sanguine, communément appelé "test ultime au lit du malade". Ce test consistait à vérifier la corrélation entre le sang du malade (receveur) et celui contenu dans la poche de sang (donneur) en principe destiné à lui être transfusé.

Les deux chambres contiguës susceptibles d'être activées simultanément par perçage d'un boudin, initialement raccordé à la poche de transfusion et introduit au moyen d'un portoir approprié, ont servi respectivement pour une réaction avec un sérum-test anti-A et une réaction avec un sérum-test anti-B sur le sang du donneur.

Les deux autres chambres, disposant chacune d'un guide pour l'introduction de tubes de prélèvement, ont servi respectivement pour une réaction avec un sérum-test anti-A et une réaction avec un sérum-test anti-B sur le sang du receveur.

Les échantillons provenaient respectivement:
- d'un tube de prélèvement sanguin d'un type couramment utilisé en médecine, comportant un moyen d'obturation perforable et déformable,
- d'une tubulure communément appelée "boudin ou nouille", solidaire des poches à transfusion et contenant un faible volume de sang.

Ils ont été mis en contact dans les chambres fermées respectives avec un sérum-test qui s'y trouvait préalablement, à savoir selon le cas du sérum-test anti-A ou du sérum-test anti-B, par un perçage exerçant une pression comme indiqué plus haut sur les aiguilles portées par les chambres réactionnelles respectives de façon à faire tomber à chaque fois au moins une goutte de sang dans la chambre réactionnelle.

Pour une meilleure réalisation des réactions d'agglutination, le dispositif a été "chaloupé" plusieurs fois, pour permettre un étalement du produit de la réaction sur presque toute la surface d'une paroi des tubes.

A la lecture, la présence d'agglutination signe la présence à la surface des hématies testées de l'antigène correspondant à l'anticorps contenu dans la chambre réactionnelle. Au contraire, un mélange homogène sans trace d'agglutination signe l'absence à la surface des hématies de l'antigène correspondant à l'anticorps contenu dans la chambre réactionnelle.

La transfusion peut être pratiquée si les résultats obtenus dans les deux chambres réservées au donneur sont équivalents aux résultats obtenus dans les deux chambres réservées au receveur.

Pour plus de sécurité et pour consigner les résultats sans risque de confusion, tout ou partie de la face du dispositif opposée à celle sur laquelle se fait la lecture des résultats peut porter une ou plusieurs étiquettes, comportant et/ou recevant des indications utiles.

## Revendications

1. Dispositif pour la réalisation de tests et/ou d'analyses de fluides, comportant au moins une chambre fermée pour tests et visualisation et au moins une aiguille pour le perçage des récipients de fluide échantillon, caractérisé en ce qu'il comprend, de bas en haut en position debout:
- un porte-tubes (A) agencé pour recevoir au moins un tube (1) partiellement ou totalement transparent, et laissant visible une paroi ou portion de paroi de celui-ci,
- au moins un tube (1) servant de chambre fermée pour test et visualisation de celui-ci, et comportant au moins un réactif de test,
- des moyens de bouchage (4) de chaque tube, comprenant en leur centre une aiguille traversante (3) ayant sa pointe vers le haut,
- une rehausse sensiblement verticale formant des parois entourant les aiguilles (3) sur une hauteur dépassant avantageusement la longueur des aiguilles au-dessus des dits moyens de bouchage (4), lesdits moyens de bouchage et lesdites aiguilles traversantes (3) étant en combinaison fonctionnelle avec des tubes de prélèvement d'échantillon de fluide à tester ou de fluide pour test, comportant un moyen d'obturation perforable et déformable, et/ou une pièce (7) formant portoir pour l'introduction de boudins souples perforables (10) contenant des fluides à tester sur au moins une aiguille, de façon à permettre leur perçage non traversant par au moins une aiguille susdite.

2. Dispositif selon la revendication 1, caractérisé en ce que ledit moyen de bouchage (4) comporte, à la base de l'aiguille traversante (3), sur la face supérieure du dit moyen de bouchage, une partie centrale surélevée ou collerette (5) entourant l'aiguille et ayant un diamètre inférieur au diamètre du dit moyen de bouchage(4).

3. Dispositif selon la revendication 1, caractérisé en ce que les aiguilles (3) comportent en outre des gaines perforables (11) enfilées sur la partie des aiguilles extérieure à ladite chambre fermée.

4. Dispositif selon l'une quelconque des revendications 1 à 3, caractérisé en ce que le portoir (7) est constitué par un bloc comportant, en position d'utilisation, un canal (8) soit en U débouchant vers le haut, soit sensiblement linéaire débouchant latéralement, ainsi qu'au moins un alésage non traversant (9) et sensiblement vertical, mettant en communication le dit canal (8) et l'extérieur sur la face inférieure du portoir.

5. Dispositif selon la revendication 1, caractérisé en ce que les tubes (1) font partie intégrante du porte-tubes (A), avantageusement sous forme d'alvéoles ménagées dans ledit porte-tubes lui-même.

6. Dispositif selon l'une quelconque des revendications 1 à 5, caractérisé en ce qu'il comporte en outre, essentiellement pour des applications médicales, en particulier dans le domaine de la transfusion sanguine, une carte ou autre support muni d'une puce, sur laquelle peuvent être mémorisées l'empreinte digitale, ainsi que d'autres informations, concernant le patient à transfuser et/ou des informations concernant la poche de sang à transfuser.

7. Procédé pour la réalisation de tests et/ou d'analyses de fluides, comprenant la mise en oeuvre d'un dispositif selon l'une quelconque des revendications 1 à 6, caractérisé en ce qu'il comporte les étapes de:
- perçage et déformation sous pression du moyen de bouchage d'un tube (6) ou d'un boudin souple (10) contenant un échantillon de fluide à tester ou de fluide pour test, pour introduction d'une quantité appropriée de prise d'essai dans au moins une chambre fermée (1) comportant un moyen de bouchage (4) muni d'une aiguille (3) de perçage et d'introduction,
- répétition de l'étape susdite jusqu'à ce que tous les réactifs de test et/ou tous les échantillons à tester ou à analyser aient été introduits dans les chambres fermées (1) respectivement concernées, et
- examen des chambres de réaction (1) concernées.

8. Utilisation d'un dispositif selon l'une quelconque des revendications 1 à 6 pour des tests et/ou analyses en biologie humaine ou vétérinaire.

9. Utilisation selon la revendication 8, en particulier dans le domaine de la transfusion sanguine, caractérisée en ce que le dispositif comporte une carte ou autre support muni d'une puce, sur laquelle peuvent être mémorisées l'empreinte digitale, ainsi que d'autres informations, concernant le patient à transfuser et/ou des informations concernant la poche de sang à transfuser.

## Patentansprüche

1. Vorrichtung zur Durchführung von Tests und/oder Analysen von Flüssigkeiten, die wenigstens eine geschlossene Kammer zum Testen und Visualisieren sowie wenigstens eine Nadel zum Durchstechen des Flüssigkeitsprobenbehälters umfasst, dadurch gekennzeichnet, dass diese von unten nach oben in aufrechter Stellung umfasst:
- einen Röhrenträger (A), der zum Aufnehmen wenigstens einer teilweise oder vollständig durchsichtigen Röhre (1) eingerichtet ist, derart, dass eine Seitenwand oder ein Teil einer Seitenwand davon sichtbar ist;
- wenigstens eine Röhre (1), die als eine geschlossene Test- und Visualisierungskammer dient, und die wenigstens ein Testreagenz umfasst;
- Verschlussmittel (4) für jede Röhre, die in ihrer Mitte eine durchgehende Nadel (3) mit einer nach oben zeigenden Spitze umfassen;
- einen etwa vertikalen Füllrahmen, der die die Nadeln (3) umgebenden Seitenwände in einer Höhe bildet, welche die Länge der Nadeln über den Verschlussmitteln (4) vorteilhafterweise überragt, wobei die Verschlussmittel und die durchgehenden Nadeln (3) in funktioneller Verbindung mit den Abzugsröhren für die Proben der zu testenden Flüssigkeit oder der Testflüssigkeit stehen, wobei diese eine durchstechbare und verformbare Verschlusseinrichtung umfassen, und/oder ein Teil (7), das eine Aufnahmevorrichtung für die Einführung der elastischen durchstechbaren Schläuche (10) ausbildet, welche die zu testenden Flüssigkeiten über wenigstens einer Nadel enthalten, dergestalt, dass diese von wenigstens einer vorstehend genannten Nadel nicht durchgehend durchstochen werden können.

2. Vorrichtung nach Anspruch 1, dadurch gekennzeichnet, dass die Verschlusseinrichtung (4) von einer Basis der durchgehenden Nadel (3) über die Oberseite der Verschlusseinrichtung ein erhöhtes mittleres Teil bzw. einen Flansch (5) umfasst, der die Nadel umgibt und dessen Durchmesser geringer ist als der Durchmesser der Verschlusseinrichtung (4).

3. Vorrichtung nach Anspruch 1, dadurch gekennzeichnet, dass die Nadeln (3) außerdem durchstechbare Hüllen (11) umfassen, die über dem Teil der Nadel außerhalb der geschlossenen Kammer aufgefädelt sind.

4. Vorrichtung nach einem der vorstehenden Ansprüche 1 bis 3, dadurch gekennzeichnet, dass die Aufnahmevorrichtung (7) durch einen Block ausgebildet ist, der, in Gebrauchsstellung, einen Kanal (8) umfasst, der entweder ein nach oben geöffnetes U bildet oder etwa linear zur Seite geöffnet ist, sowie wenigstens eine nicht durchgehende und etwa vertikale Bohrung (9), wobei der Kanal (8) und die Außenseite an der Unterseite der Aufnahmevorrichtung verbunden sind.

5. Vorrichtung nach Anspruch 1, dadurch gekennzeichnet, dass die Röhren (1) einen integralen Teil des Röhrenträgers (A) bilden, vorteilhafterweise in Form von ausgesparten Zellen in dem Röhrenträger selbst.

6. Vorrichtung nach einem der Ansprüche 1 bis 5, dadurch gekennzeichnet, dass diese außerdem, im wesentlichen für medizinische Anwendungen insbesondere auf dem Gebiet der Bluttransfusion, eine Karte oder einen anderen mit einem Chip versehenen Träger umfasst, auf welcher/welchem Fingerabdrücke gespeichert sein können sowie andere Informationen über den Transfusionspatienten und/oder Informationen über den Transfusionsblutbeutel.

7. Verfahren zur Durchführung von Tests und/oder Analysen von Flüssigkeiten, das die Verwendung einer Vorrichtung nach einem der Ansprüche 1 bis 6 umfasst, dadurch gekennzeichnet, dass dieses die folgenden Schritte umfasst:
- Durchstechen und Verformen unter Druck der Verschlussmittel einer Röhre (6) oder eines elastischen Schlauches (10), der eine Probe der zu testenden Flüssigkeiten oder der Testflüssigkeiten enthält, zum Einführen einer geeigneten Probenmenge in wenigstens eine geschlossene Kammer (1), die eine Verschlusseinrichtung (4) umfasst, die mit einer Nadel (3) zum Durchstechen und zum Einführen versehen ist,
- Wiederholung des vorstehenden Schrittes solange bis alle Testreagenzien und/oder alle Proben zum Testen oder zum Analysieren in jede der entsprechenden geschlossenen Kammern (1) eingeführt worden sind,
- Untersuchung der entsprechenden Reaktionskammern (1).

8. Verwendung einer Vorrichtung nach einem der Ansprüche 1 bis 6 zum Testen und/oder Analysieren in der Human- oder Veterinärbiologie.

9. Verwendung nach Anspruch 8, insbesondere auf dem Gebiet der Bluttransfusion, dadurch gekennzeichnet, dass die Vorrichtung eine Karte oder einen anderen mit einem Chip versehenen Träger umfasst, auf welcher/welchem Fingerabdrücke gespeichert sein können, so wie andere Informationen über den Transfusionspatienten und/oder Informationen über den Transfusionsblutbeutel.

## Claims

1. Device for carrying out tests and/or analyses of fluids, comprising at least one closed chamber for tests and observation and at least one needle for perforating sample fluid receptacles, characterized in that it comprises, from its base up and in an upright position:
- a tube holder (A) for receiving at least one partially or totally transparent tube (1), and leaving at least one of its walls or wall portions visible;
- at least one tube (1) serving as a closed chamber for test and for observation of the results thereof, and comprising at least one test reagent;
- closure means (4) of each tube, comprising in their center a needle extending therethrough (3) with its point facing upwards;
- a substantially vertical raised portion forming walls surrounding the needles (3) and advantageously higher than the distance of the needles by which they project above said closure means (4), said closure means and said throughgoing needles (3) being in functional combination with fluid sample tubes or test fluid tubes, comprising a perforable and deformable closure means and/or a member (7) forming a housing for the introduction of perforable flexible hoses (10) containing fluids to be tested on at least one needle, so as to permit their non-throughgoing perforation by at least one of the above needles.

2. Device according to Claim 1, characterized in that said closure means (4) comprises, at the base of the throughgoing needle (3), on the upper surface of said closure means, a central raised portion or flange (5) surrounding the needle and having a diameter smaller than the diameter of said closure means (4).

3. Device according to Claim 1, characterized in that the needles (3) also comprise perforable sheaths (11) placed on the part of the needles outside said closed chamber.

4. Device according to any one of Claims 1 to 3, characterized in that the housing (7) consists of a unit comprising, in the position of use, a channel (8) which is either U-shaped opening into the top, or substantially linear which opens laterally, as well as at least one non-throughgoing and substantially vertical bore (9), putting into communication said channel (8) and the exterior on the lower surface of the housing.

5. Device according to Claim 1, characterized in that the tubes (1) form an integral part of the tube holder (A), advantageously in the form of cells created in said tube holder itself.

6. Device according to any one of Claims 1 to 5, characterized in that it also comprises, essentially for medical applications, particularly in the area of blood transfusion, a card or other support provided with a chip, on which may be stored the digital imprint and other data concerning the patient to be transfused and/or data concerning the bag of blood to be transfused.

7. Process for carrying out tests and/or analyses of fluids, comprising the use of a device according to any one of Claims 1 to 6, characterized in that it consists of the steps of:
- perforating and deforming under pressure the closure means of a tube (6) or of a flexible hose (10) containing a sample of fluid to be tested or test fluid, for introduction of an appropriate amount of test sample into at least one closed chamber (1) comprising a closure means (4) equipped with a needle (3) for perforation and introduction;
- repeating the aforementioned step until all the test reagents and/or all the samples to be tested or analyzed have been introduced into the respective closed chambers (1); and
- examining the respective reaction chambers (1).

8. Use of a device according to any one of Claims 1 to 6 for tests and/or analyses in human or veterinary biology.

9. Use according to Claim 8, particularly in the area of blood transfusion, characterized in that the device comprises a card or other support having a chip on which may be stored the digital imprint and other data concerning the patient to be transfused and/or data concerning the bag of blood to be transfused.
